# NEW EUROPEAN PATENT SPECIFICATION

(11) **EP 0 782 644 B2**
(45) Date of publication and mention of the opposition decision: **19.04.2006**
(45) Mention of the grant of the patent: 07.11.2001
(21) Application number: 95930795.0
(22) Date of filing: 04.08.1995
(51) Int. Cl.: D21H 11/08, D21H 21/20, A61F 13/15

(54) **TISSUE OR PAPER TOWEL COMPRISING WET-RESILIENT WEBS**
PAPIERTUCH MIT IM NASSEN ZUSTAND FEDERNDE FASERSTOFFBAHN
SERVIETTES EN PAPIER COMPRENANT DES BANDES PRESENTANT UNE ELASTICITE A L'ETAT HUMIDE

(30) Priority: 21.09.1994 US 310186
(43) Date of publication of application: 09.07.1997
(73) Proprietor: KIMBERLY-CLARK WORLDWIDE, INC., Neenah, WI 54956 (US)
(72) Inventor: CHEN, Fung-jou, Appleton, WI 54915 (US); KAMPS, Richard, Joseph, Wrightstown, WI 54180 (US); BURAZIN, Mark, Alan, Appleton, WI 54915 (US); HOLLENBERG, David, Henry, Neenah, WI 54956 (US)
(74) Representative: Davies, Christopher Robert
(86) International application number: PCT/US1995/009942
(87) International publication number: WO 1996/009435

(56) References cited:
- EP-A- 0 568 404
- EP-A- 0 631 014
- WO-A-94/10956
- US-A- 3 301 746
- US-A- 4 120 747
- US-A- 4 981 557
- US-A- 5 160 789
- US-A- 5 223 096

## Description

### Background of the Invention

In the manufacture of absorbent paper products such as facial tissue, bath tissue, paper towels, napkins and the like, many different sheet properties influence the performance of the particular product being made. Softness, strength, absorbency, bulk and the like are often the subject of improvements. However, a property of tissue-related products is that when wetted and crumpled in the hand, they essentially collapse into a dense w t mass. Stated another way, such tissue products have a low wet compressive modulus and low wet resiliency. These properties are undesirable for such products when used to wipe up liquids because, once saturated they become useless.

From US-A-4,981,557, temporary wet-strength resins are known having a molecular weight from about 200,000. These resins can be comprised in a paper product.

Form US-A-4,129,518, uncreped absorbent structures in which the fibers are immobilize with wet resistant bonds are known.

Those documents do not suggest the combination of a wet-strength agent with a structure comprising wet resilient fibers.

Hence there is a need for an improved paper sheet useful as a tissue, towel, and the like, which substantially retains its integrity when wetted and acts more like a sponge than prior tissue products.

### Summary of the Invention

It has now been discovered that by property combining certain natural paper-making fibers with a wet strength resin in what is commonly referred to as an uncreped throughdrying process, low density absorbent webs can be produced which exhibit remarkable wet resiliency. These webs contain wet resilient natural fibers which have been bonded together with water resistant bonds, resulting in a structure which resists structural collapse, gathering or dimensional change when wet, thus exhibiting a stable capillary structure advantageous for absorbent products. The low density absorbent structures have a density of about 0.3 g/cm³ or less and are uncreped. These absorbent wet resilient structures are useful as tissues, paper towels, napkins and the like, an also as a component In other absorbent products such as disposable diapers, incontinence garments, training pants, feminine pads, poultry pads, and the like. The structures of such other absorbent products are well known in the art. In such applications, or in other applications In which fluids are contained, entrapped, or transported, the wet resilient structures of this invention can function as fluid surge webs, fluid distribution webs, absorbent cores or composites, and the like.

High yield pulp fibers contain a high level of lignin, which is believed to impart wet resilience to the fibers. The wet strength resin bonds (immobilizes) the wet-resilient fibers into a sheet structure conforming to the contour of the throughdrying fabric. As the sheet is dried, the bonds formed by the wet strength agent are cured to form wet-resistant bonds which together provide a sheet that is highly resilient when wet. This property is retained In the sheet because, In the case of an uncreped throughdried process, here is no creping step or other subsequent step, to disrupt the bonds that have been formed.

The invention resides in a tissue or paper towel according to claim 1. Wet-resilient natural fibers include high yield pulp fibers (further discussed below), flax, milkweed, abaca, hemp, cotton or any of the like that are naturally wet resilient or any wood pulp fibers that are chemically or physically modified, e.g. crosslinked or curled, that have the capability to recover after deformation in the wet state, as opposed to non-resilient fibers which remain deformed and do not recover after deformation in the wet state. Wet-resistant bonds are fiber-to-fiber bond sites that are resistant to disruption in the wet state resulting in a wet tensile strength:dry tensile strength ratio of 0.1 or greater.

As used herein, "high yield pulp fibers" are those papermaking fibers produced by pulping processes providing a yield of about 65 percent or greater, more specifically about 75 percent or greater, and still more specifically from about 75 to about 95 percent. Such pulping processes include bleached chemithermomechanical pulp (BCTMP), chemithermomechanical pulp (CTMP) pressure/pressure thermomechanical pulp (PTMP), thermomechanical pulp (TMP), thermomechanical chemical pulp (TMCP), high yield suiphite pulps, and high yield kraft pulps, all of which leave the resulting fibers with high levels of lignin. The preferred high yield pulp fibers are characterized by being comprised of comparatively whole, relatively undamaged tracheids, high freeness (over 250 CSF), and low fines content (less than 25 percent by the Britt jar test).

The amount of high yield pulp fibers in the sheet is at least about 30 dry weight percent or greater, still more specifically about 50 dry weight percent or greater, and up to 100 percent. For layered sheets, these same amounts can be applied to one or more of the individual layers. Because high yield pulp fibers are generally less soft than other papermaking fibers, It is advantageous to incorporate them into the middle of the final product, such as placing them in the center layer of a three-layered sheet or, in the case of a two-ply product, placing them in the inwardly-facing layers of each of the two plies.

The products of this invention have low densities (high bulks). In general, the density of the products of this invention can be about 0.3 gram per cubic centimeter or less, more specifically about 0.15 gram or less, still more specifically about 0.1 gram per cubic centimeter or less. It is believed to be important that the absorbent structure, once formed, be dried without substantially reducing the number of wet-resilient interfiber bonds. Throughdrying, which is a common method for drying tissues and towels, is a preferred method of preserving the structure. Absorbent structures made by wet laying followed by throughdrying typically have a density of about 0.1 gram per cubic centimeter, whereas airlaid structures normally used for diaper fluff typically have densities of about 0.05 gram per cubic centimeter. All of such structures are within the scope of this invention.

An integral part of the Invention is the material used to immobilize the bonds between the fibers in the wet state. Typically the means by which fibers are held together in paper and tissue products invoive hydrogen and sometimes combinations of hydrogen bonds and covalent and/or ionic bonds. In the present invention, it is important to provide a material that will allow bonding of fibers in such a way as to immobilize the fiber to fiber bond points and make them resistant to disruption in the wet state. In this instance the wet state usually will mean when the product is exposed to water or other aqueous solutions, but could also mean exposure to body fluids such as urine, blood, mucus, menses, lymph and other body exudates.

There are a number of materials commonly used in the paper industry to impart wet strength to paper and board that are applicable to this invention. These materials are known in the art as wet strength agents and are commercially available from a wide variety of sources. Any material that when added to a paper ortissue results in providing a tissue or paper with a wet strength:dry strength ratio in excess of 0.1 will, for purposes of this invention, be termed a wet strength agent. Typically these materials are termed either as permanent wet strength agents or as "temporary" wet strength agents. For the purposes of differentiating permanent from temporary wet strength, permanent will be defined as those resins which, when incorporated into paper or tissue products, will provide a product that retains more than 50% of its original wet strength after exposure to water for a period of at least five minutes. Temporary wet strength agents are those which show less than 50% of their original wet strength after exposure to water for five minutes. Both classes of material find application in the present invention. The amount of wet strength agent added to the pulp fibers can be at least about 0.1 dry weight percent, more specifically about 0.2 dry weight percent or greater, and still more specifically from about 0.1 to about 3 dry weight percent based on the dry weight of the fibers.

Permanent wet strength agents will provide a more or less long-term wet resilience to the structure. This type of structure would find application in products that would require long-term wet resilience such as in paper towels and in many absorbent consumer products. In contrast, the temporary wet strength agents would provide structures that had low density and high resilience, but would not provide a structure that had long-term resistance to exposure to water or body fluids. While the structure would have good integrity initially, after a period of time the structure would begin to lose its wet resilience. This property can be used to some advantage in providing materials that are highly absorbent when initially wet, butwhich after a period of time lose their integrity. This property could be used in providing "flushable" products. The mechanism by which the wet strength is generated has little influence on the products of this Invention as long as the essential property of generating water-resistant bonding at the fiber/fiber bond points is obtained.

The permanent wet strength agents that are of utility in the present invention are typically water soluble, cationic oligomeric or polymeric resins that are capable of either crosslinking with themselves (homocrosslinking) or with the cellulose or other constituent of the wood fiber. The most widely-used materials for this purpose are the class of polymer known as polyamide-polyamine-epichlorohydrin (PAE) type resins. These materials have been described in patents issued to Keim (U.S.-A-3,700,623 and 3,772,076) and are sold by Heroules, Inc., Wilmington, Delaware, as Kymene 557H. Related materials are marketed by Henkel Chemical Co., Charlotte, North Carolina and Georgia-Pacific Resins, Inc., Atlanta, Georgia.

Polyamide-epichlorohydrin resins are also useful as bonding resins in this invention. Materials developed by Monsanto and marketed under the Santo Res label are base-activated polyamide-epichiorohydrin resins that can be used in the present invention. These materials are described in patents issued to Petrovich (U.S.-A-3,885,158; U.S.-A-3,899,388; U.S.-A-4,129,528 and U.S.-A-4,147,586) and van Eenam (U.S.-A-4,222,921). Although they are not as commonly used in consumer products, polyethylenimine resins are also suitable for immobilizing the bond points in the products of this invention. Another class of permanent-type wet strength agents are exemplified by the aminoplast resins obtained by reaction of formaldehyde with melamine or urea.

The temporary wet strength resins that can be used in connection with this invention include, but are not limited to, those resins that have been developed by American Cyanamid and are marketed under the name Parez 631 NC (now available from Cytec Industries, West Paterson, New Jersey. This and similar resins are described in U. S.-A-3,556,932 to Coscia et al. and 3,556,933 to Williams et al. Other temporary wet strength agents that should find application in this invention include modified starches such as those available from National Starch and marketed as Co-Bond 1000. it is believed that these and related starches are covered by U.S.-A-4,675,394 to Solarek et al. Derivatized diaidehyde starches, such as described in Japanese Kokai Tokkyo Koho JP-A-03,185,197, should also find application as useful materia is for providing temporary wet strength. It is also expected that othertemporary wet strength materials such as those described in U.S.-A-4,981,557; U.S.-A-5,008,344 and U.S.-A-5,085,736 to Bjorkquist would be of use in this invention. With respect to the classes and the types of wet strength resins listed, it should be understood that this listing is simply to provide examples and thatthis is neither meant to exclude othertypes of wet strength resins, nor is it meant to limit the scope of this invention.

Although wet strength agents as described above find particular advantage for use in connection with in this invention, other types of bonding agents can also be used to provide the necessary wet resiliency. They can be applied at the wet end or applied by spraying or printing, etc. after the web is formed or after it Is dried.

It has been observed that the products of this invention exhibit significantly greater wet resiliency than other similar products. For example, when the products of this invention are saturated with water and crumpled in one's hand into a ball about the size of a golfball, and thereafter released, they quickly open up to mostly uncrumple themselves. By contrast, current commercially-available products such as bath tissues and paper towels remain substantially wadded up in a wet ball. In order to objectively quantify this property, several parameters are used. They are the wet tensile strength:dry tensile strength ratio (wet;dry ratio), the Wet Wrinkle Recovery Test, and wet compressive resiliency, all of which are described below. These parameters can be used to define absorbent structures of this invention alternatively or in combination.

The wet:dry ratio is simply the ratio of the wet tensile strength divided by the dry tensile strength. It can be expressed using the machine direction (MD) tensile strengths, the cross-machine direction (CD) tensile strengths, or the geometric mean tensile strengths (GMT). Absorbent structures of this invention have a GMT wet:dry ratio of 0.1 or greater, more specifically about 0.2 or greater, still more specifically about 0.35 or greater, and still more specifically about 0.5 or greater.

The Wet Wrinkle Recovery Test Is a slight modification of AATCC Test Method 66-1990 taken from the Technical Manual of the American Association of Textile Chemists and Coiorists (1992), page 99. The modification is to first wet the samples before carrying out the method. This is done by soaking the samples in water containing 0.01 percent TRITON X-100 for five minutes before testing. Sample preparation is carried out at 22.8°C (73°F.) and 50 percent relative humidity. The sample is gently removed from the water with a tweezers, drained by pressing between two pieces of blotter paper with 325 grams of weight, and placed in the sample holder to be tested as with the dry wrinkle recovery test method. The test measures the highest recovery angle of the sample being tested (in any direction, including the machine direction and the cross-machine direction), with 180° representing total recovery. The percent wet wrinkle recovery is the measured recovery angle divided by 180°, multiplied by 100. Absorbent structures of this invention can exhibit a percent wet wrinkle recovery of about 60 percent or greater, more specifically about 70 percent or greater, and still more specifically about 80 percent or greater.

Wet compressive resiliency of the new materials can be demonstrated using a materials property procedure that encompasses both wet and dry characteristics. A programmable strength measurement device is used in compression mode to impart a specified series of compression cycles to an initially dry, conditioned sample, after which the sample is carefully moistened in a specified manner and subjected to the same sequence of compression cycles, while the comparison of wet and dry properties is of general interest, the most Important information from this test concerns the wet properties. The initial testing of the dry sample can be viewed as a conditioning step.

Each test specimen consists of a stack of two or more conditioned (24 hours @ 50% RH, 22.8°C (73° F)) dry sample sheets cut to 6,35 cm (2.5") squares, providing a stack mass preferably between 0.2 and 0,6 g. The test sequence begins with compression of the dry sample to 0.17 kPa (0.025 psi) to obtain an initial thickness (cycle A), then two repetitions of loading up to 13.79 kPa (2 psi) followed by unloading (cycles B and C). Finally, the sample is again compressed to 0.17 kPa (0.025 psi) to obtain à final thickness (cycle D). (Details of the procedure, including compression speeds, are given below). Following the treatment of the dry sample, moisture is applied uniformly to the sample using a fine mist of deionized water to bring the moisture ratio (g water/g dry fiber) to approximately 1.1. This is done by applying 95-110% added moisture, based on the conditioned sample mass. This puts typical cellulosic materials in a moisture range where physical properties are relatively insensitive to moisture content (e.g., the sensitivity is much less than it is for moisture ratios less than 70%). The moistened sample is then placed in the test device and the compression cycles are repeated.

Three measures of wet resiliency are considered which are relatively, insensitive to the number of sample layers used in the stack. The first measure is the bulk of the wet sample at 13,79 kPa (2 psi.). This is referred to as the "Compressed Bulk". The second measure is termed "Springback Ratio", which is the ratio of the moist samole thickness at 0.17 kPa (0.025 psi) at the end of the compression test (cycle D) to the thickness of the moist sample at 0.17 kPa (0.025 psi) measured at the beginning of the test (cycle A). The third measure is the "Loading Energy Ratio", which is the ratio of loading energy in the second compression to 13.79 kPa (2 psi) (cycle C) to that of the first compression to 13.79 kPa (2 psi) (cycle B) during the sequence described above, for a wetted sample. When load is plotted as a function of thickness; Loading Energy is the area under the curve as the sample goes from an unloaded state to the peak load of that cycle. For a purely elastic material, the springback and loading energy ratio would be unity. We have found that the three measures described here are relatively independent of the numberof layers in the stack and serve as useful measures of wet resiliency. Also referred to herein is the Compression Ratio, which is defined as the ratio of moistened sample thickness at peak load in the first compression cycle to 13.79 kPa (2 psi) to the initial moistened thickness at 0.17 kPa (0.025 psi.).

Absorbent structures of this invention can exhibit one or more of the foregoing properties. More specifically, the absorbent structures of this invention have a Compression Ratio of about 0.7 or less, more specifically about 0.6 or less, and still more specifically about 0.5 or less. Also, they have a Springback Ratio of about 0.75 or greater, more specifically about 0.8 or greater, more specifically about 0.85 or greater, and still more specifically about 0.9 or greater. The Loading Energy Ratio can be about 0.7 or greater, more specircally about 0.8 or greater

In carrying out the measurements of the wet compression recovery, samples should be conditioned for at least 24 hours under TAPPI conditions (50% RH, 22.8°C (73°F.)). Specimens are die cut to 6,35 cm x 6,35 cm (2.5" x 2.5°) squares. Conditioned sample weight should be near 0.4 g, if possible, and within the range of 0.25 to 0.6 g for meaningful comparisons. The target mass of 0.4 g is achieved by using a stack of 2 or more sheets if the sheet basis weight is less than 65 g·m⁻² (65 gsm). For example, for nominal 30 g·m⁻² (30 gsm) sheets, a stack of 3 sheets will generally be near 0.4 g total mass.

Compression measurements are performed using an instron 4502 Universal Testing Machine interfaced with a 826 PC computer running instron Series XII software (1989 issue) and Version 2 firmware. A 100 kN load cell is used with 5,715 cm (2.25") diameter circular platens for sample compression. The lower platen has a ball bearing assembly to allow exact alignment of the platens. The lower platen is locked in place while under load 133.4, 444.8 N (30-100 Ibf) by the upper platen to ensure parallel surfaces The upper platen must also be locked in place with the standard ring nut to eliminate play in the upper platen as load Is applied.

Following at least one hour of warm-up after start-up, the instrument control panel is used to set the extensionometer to zero distance while the platens are in contact (at a load of 4.536 kg - 13,608 kg (10-30 Ib). With the upper platen freely suspended, the calibrated load cell is balanced to give a zero reading. The extensionometer and load cell should be periodically checked to prevent baseline drift (shirting of the zero points). Measurements must be performed in a controlled humidity and temperature environment, according to TAPPI specifications (50% ± 2% rh and 22.8°C (73° F). The upper platen is then raised to a height of 0,508 cm (0.2 in) and control of the Instron is transferred to the computer.

Using the Instron Series XII Cyclic Test software, an instrument sequence is established with 7 markers (discrete events) composed of 3 cyclic blocks (instructions sets) in the following order:
Marker 1: Block 1
Marker 2: Block 2
Marker 3: Block 3
Marker 4: Block 2
Marker 5: Block 3
Marker 6: Block 1
Marker 7: Block 3.

Block 1 instructs the crosshead to descend at 3,81 cm/min (1.5. in./min), until a load of 0,045 kg (0.1 lb), is applied (the Instron setting is -0.1 lb., since compression is defined as negative force). Control is by displacement.
When the targeted load is reached, the applied load is reduced to zero.

Block 2 directs that the crosshead range from an applied load of 0,023 kg (0.05 lb), to a peak of 3,629 kg (B lb.) then back to 0,023 kg (0.05 lb), at a speed of 1,02 cm/min (0.4 in./min), Using the Instron software, the control mode is displacement, the limit type is load, the first level is - 0,023 kg (-0.05 lb.), the second level is - 3,629 kg (-8 lb), the dwell time is 0 sec., and the number of transitions is 2 (compression, then relaxation); "no action" is specified for the end of the block.

Block 3 uses displacement control and limit type to simply raise the crosshead to 0,508 cm (0.2 in) at a speed of 10,16 cm/min (4 in./min), with 0 dwell time. Other Instron software settings are 0 in first level, 0.2 in second level, 1 transition, and "no action" at the end of the block.

When executed in the order given above (Markers 1-7), the instron sequence compresses the sample to 0.17 kPa (0.025 psi (0.1 lbf)), relaxes, then compresses to 13.79 kPa (2 psi (8 lbs.)), followed by decompression and a crosshead rise to 0,508 cm (0.2 in.), then compress the sample again to 13,79 kPa (2 psi), relaxes, lifts the crosshead to 0.508 cm (0.2 in), compresses again to 0,17 kPa (0.025 psi) 0,44 N ((0.1 lbf)), and then raises the crosshead. Data logging should be performed at Intervals no greater than every 0,051 cm (0.02") or 0,181 kg (0.4 lb) (whichever comes first) for Block 2.2) and for intervals no greater than 0.005 kg (0.01 lb.) for Block 1. Preferably, data logging is performed every 0,0018 kg (0.004 Ib.) in Block 1 and every 0,0227 kg (0.05 Ib.) or 0,013 cm (0.005 in.) (whichever comes first) in Block 2.

The results output of the Seriex XII software is set to provide extension (thickness) at peak loads for Markers 1, 2, 4 and 6 (at each 0.17 kPa and 13.79 kPa (0.025 and 2.0 psi) peak load), the loading energy for Markers 2 and 4 (the two compressions to 13.79 kPa (2.0 psi) previously termed cycles B and C, respectively), the ratio of the two loading energies (second cycie/first cycle), and the ratio of final thickness to initial thickness (ratio of thickness at last to first 0.17 kPa (0.025 psi) compression). Load versus thickness results are plotted on the screen during execution of Blocks 1 and 2.

In performing a measurement, the dry, conditioned sample is centered on the lower platen and the test is initiated. Following completion of the sequence, the sample is immediately removed and moisture (deionized water at 22.2°C-22.8°C (72-73° F)) Is applied. Moisture is applied uniformly with a fine mist to reach a moist sample mass of approximately 2.0 times the initial sample mass (95-110% added moisture is applied, preferably 100% added moisture, based on conditioned sample mass ; this level of moisture should yield an absolute moisture ratio between 1.1 and 1.3 g. water/g, oven dry fiber - with oven dry referring to drying for at least 30 minutes in an oven at 105° C). The mist should be applied uniformly to separated sheets (for stacks of more than 1 sheet), with spray applied to both front and back of each sheet to ensure uniform moisture application. This can be achieved using a conventional piastic spray bottle, with a container or other barrier blocking most of the spray, allowing only about the upper 10-20% of the spray envelope - a fine mist - to approach the sample. The spray source should be at least 10" away from the sample during spray application. In general, care must be applied to ensure that the sample is uniformly moistened by a fine spray. The sample must be weighed several times during the process of applying moisture to reach the targeted moisture content. No more than three minutes should elapse between the completion of the compression test on the dry sample and the completion of moisture application. Allow 45-60 seconds from the final application of spray to the beginning of the subsequent compression test to provide time for intemal wicking and absorption of the spray. Between three and four minutes will elapse between the completion of the dry compression sequence and initiation of the wet compression sequence.

Once the desired mass range has been reached, as indicated by a digital balance, the sample is centered on the lower Instron platen and the test sequence is initiated. Following the measurement, the sample is placed in a 105° C-oven for drying, and the oven dry weight will be recorded later (sample should be allowed to dry for 30-60 minutes, after which the dry weight is measured).

Note that creep recovery can occur between the two compression cycles to 13.79 kPa (2 psi) so the time between the cycles may be important. For the instrument settings used in these instron tests, there is a 30 second period (± 4 sec.) between the beginning of compression during the two cycles to 13.79 kPa (2 psi). The beginning of compression is defined as the point at which the load cell reading exceeds 0.014 kg (0.03 Ib). Likewise, there is a 5-8 second interval between the beginning of compression in the first thickness measurement (ramp to 0.17 kPa (0.025 psi)) and the beginning of the subsequent compression cycle to 13.79 kPa (2 psi). The interval between the beginning of the second compression cycle to 13.79 kPa (2 psi) and the beginning of compression for the final thickness measurement is approximately 20 seconds.

### Brief Description of the Drawing

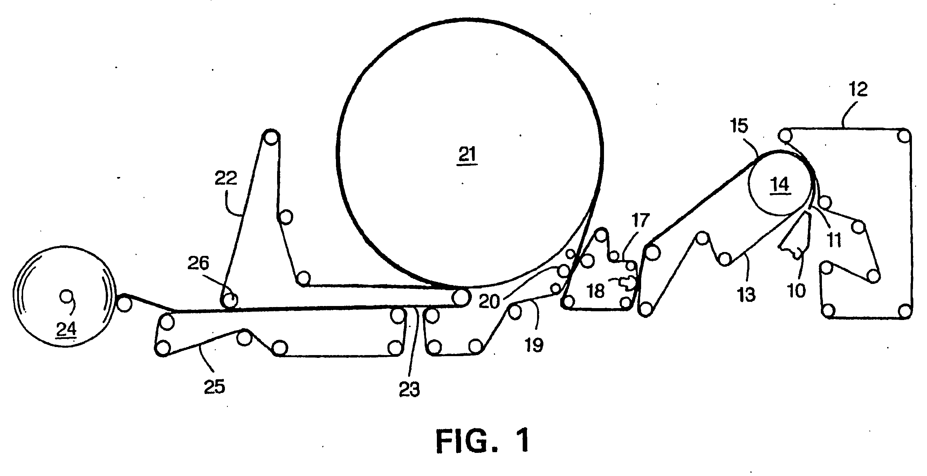

Figure 1 is a schematic diagram of an uncreped throughdried papermaking process useful for making wet resilient absorbent structures of this invention.

### Detailed Description of the Drawing

Referring to Figure 1, shown is a method for making throughdried paper sheets. (For simplicity, the various tensioning rolls schematically used to define the several fabric runs are shown but not numbered. It will be appreciated that variations from the apparatus and method illustrated in Figure 1 can be made without departing from the scope of the invention). Shown is a twin wire former having a layered papermaking headbox 10 which injects or deposits a stream 11 of an aqueous suspension of papermaking fibers onto the forming fabric 13 which serves to support and carry the newly-formed wet web downstream in the process as the web is partially dewatered to a consistency of abo Ot 10 dry weight percent. Additional dewatering of the wet web can be carried out, such as by vacuum suction, while the wet web is supported by the forming fabric.

The wet web is then transferred from the forming fabric to a transfer fabric 17 traveling at a slower speed than the forming fabric in order to impart increased stretch into the web. Transfer is preferably carried out with the assistance of a vacuum shoe 18 and a fixed gap or space between the forming fabric and the transfer fabric or a kiss transfer to avoid compression of the wet web.

The web is then transferred from the transfer fabric to the throughdrying fabric 19 with the aid of a vacuum transfer roll 20 or a vacuum transfer shoe, optionally again using a fixed gap transfer as previously described. The throughdrying fabric can be traveling at about the same speed or a different speed relative to the transfer fabric. If desired, the throughdrying fabric can be run at a slower speed to further enhance stretch. Transfer is preferably carried out with vacuum assistance to ensure deformation of the sheet to conform to the throughdrying fabric, thus yielding desired bulk and appearance.

The level of vacuum used for the web transfers can be from about(3 to about 15 inches of mercury) 75 to about 380 millimeters of mercury, preferably about(5 inches) 125 millimeters of mercury. The vacuum shoe (negative pressure) can be supplemented or replaced by the use of positive pressure from the opposite side of the web to blow the web onto the next fabric in addition to or as a replacement for sucking it onto the next fabric with vacuum. Also, a vacuum roll or rolls can be used to replace the vacuum shoe(s).

While supported by the throughdrying fabric, the web is final dried to a consistency of about 94 percent or greater by the throughdryer 21 and thereafter transferred to a carrier fabric 22. The dried basesheet 23 is transported to the reel 24 using carrier fabric 22 and an optional carrier fabric 25. An optional pressurized tuming roll 26 can be used to facilitate transfer of the web from carrier fabric 22 to fabric 25. Suitable carrier fabrics for this purpose are Albany international 84M or 94M and Asten 959 or 937, all of which are relatively smooth fabrics having a fine pattern. Although not shown, reel calendering or subsequent off-line calendering can be used to improve the smoothness and softness of the basesheet.

### Examples

### Examples 1-4

In order to illustrate a method of making absorbent structures of this invention, paper sheets were produced using non-wet resilient northern softwood kraft fibers (NSWK), with and without a wet strength agent 9,072 kg/ton ((20 Ibs/ton) Kymene), and wet resilient fibers (spruce BCTMP), with and without a wet strength agent 9,072 kg/ton (20 lbs/ton) Kymene), using an uncreped throughdried process as described in Figure 1.

The fiber was pulped at 4% consistency in the hydropulper for 30 minutes. The fiber was pumped into a stock chest and diluted to 1.0% consistency. 20#/ton of Kymene 557-LX was added to the stock chest and allowed to mix for 30 minutes. A single-layer, blended sheet of 30 gsm dry weight was formed on an Albany 94M forming fabric and dewatered with 127 millimeters (5 inches) of mercury vacuum. The forming fabric was traveling at .35 meters per second (69 fpm). The sheet was transferred at a 15% rush transfer to a Lindsay 952-S05 transfer fabric traveling at 30 meters per second (60 fpm). The vacuum in the transfer between the forming fabric and transfer fabric was 254 millimeters (10 inches) of mercury.

The sheet was vacuum transferred at 12 inches (305 millimeters) of mercury to a throughdryer fabric (Lindsay T116-1) traveling at the same speed as the transferfabric, .30 meters per second (60 fpm). The sheet and throughdryer fabric traveled over a fourth vacuum at (12 inches) 305 millimeters of mercury just prior to entering a Honeycomb throughdryer operating at 93°C (200°F) and dried to a final dryness of 94-98% consistency.

The sheets were aged for over 5 days at less than 50% humidity at 21°C (70°F). The sheets were tested for physical characteristics in a controlled environment of 50% ± 2% humidity and 23°C ± 1°. The wet and dry strength were Instron tested with a(3-inch) 7.62 cm sample width 10.16 cm (4-inch) jaw span at 25.4 cm/min (10 in/min) crosshead speed. Caliper was measured with the TMI tester at 2.0 kPa (0.289 psi).

| | Example 1 | Example 2 | Example 3 | Example 4 |
|---|---|---|---|---|
| Furnish | MSWK | MWSK | Spruce | Spruce |
| | | | BCTMP | BCTMP |
| Eymene | 0 | 20#/ton | 0 | 20/ton |
| 5 MD grams dry | 1592 | 2761 | 1678 | 2257 |
| MD Z stretch dry | 7.6 | 10.0 | 1.8 | 1.8 |
| CD grame dry | 1671 | 2459 | 1540 | 1872 |
| CD % stretch dry | 5.0 | 5.7 | 3.5 | 3.2 |
| GMT grams dry | 1631 | 2606 | 1608 | 2056 |
| MD grams wet | 106 | 892 | 49 | 826 |
| MD % stretch wet | 13.4 | 8.8 | 6.8 | 3.2 |
| CD grams wet | 71 | 715 | 47 | 759 |
| CD % stretch wet | 9.0 | 5.3 | 5.5 | 3.2 |
| GMT grams wet | 87 | 798 | 48 | 792 |
| HD % wet/dry | 6.6 | 32.3 | 2.9 | 36.6 |
| CD % wet/dry | 6.2 | 29.1 | 3.1 | 40.5 |
| GMT % wet/dry | 5.3 | 30.6 | 3.0 | 38.5 |
| Basis Weight(g/m) | 31.7 | 32.2 | 32,4 | 32.7 |
| 1-Sheet TMI mm | .602 | .605 | .630 | .602 |
| 10-Sheet TMI mm | 3.34 | 3.68 | 3.91 | 3.95 |
| Density,(g/cc) | .053 | .053 | ..051 | .054 |
| Bulk(cc/g) | 19.0 | 18.8 | 19.4 | 18.4 |
| Absorbency @ (0.75 psi) | | | | |
| Merzncal g/g | 7.6 | 8.7 | 102 | 10.1 |
| 45° g/g | 7.1 | 7.6 | 9.7 | 9.3 |
| Percent Wet Wrinkle Recovery | 34.4 | 52.7 | 35.0 | 81.6 |

As shown, Example 4 (this invention) exhibited substantially greater wet resiliency, as measured by the Wet Wrinkle Recovery Test, than the other three samples. In addition, Example 4 also showed a high wet:dry ratio.

### Examples 5-8

Further examples were carried out similar to those described in Examples 1-4, but for the purpose of exploring the basis weight effect on a bulky, absorbent, wet resilient structure. Four basis weight levels of 30, 24, 18 and 13 gsm of 100% Spruce BCTMP with 20#/ton Kymene were produced.

The fiber was pulped at 4% consistency in the hydropulper for 30 minutes. The fiber was pumped into a stock chest and diluted to 1.0% consistency. 20#/ton of Kymene 557 LX was added to the stock chest and allowed to mix for 30 minutes. A single-layer, blended sheet was formed on an Albany 94M forming fabric and dewatered with (4 inches) 102 millimeters of mercury vacuum. The forming fabric was traveling at (69 fpm) .35 meters per second. The sheet was transferred at a 15% rush transfer to a Lindsay 952-S05 transfer fabric traveling at (60 fpm) .30 meters per second. The vacuum in the transfer between the forming fabric and transfer fabric was (7 inches) 178 millimeters of mercury. The 13 gm⁻² (13 gsm) sample was produced without a rush transfer, the forming fabric was traveling at (60 fpm) .30 meters per second , the same as the transfer fabric and throughdryer fabric.

The sheet was vacuum transferred at (10 inches) 254 millimeters of mercury to a throughdryer fabric (Lindsay T116-1) traveling at the same speed as the transfer fabric, (60 fpm) .30 meters per second . The sheet and throughdryer fabric traveled over a fourth vacuum at (11 inches) 279 millimeters of mercury just prior to entering a Honeycomb throughdryer operating at (260°F) 127°C and dried to a final dryness of 94-98% consistency.

The sheets were aged for over 5 days at less than 50% humidity at (70°F) 21 °C. The sheets were tested for physical characteristics in a controlled environment of 50% ± 2% humidity and 23°C ± 1°. The wet and dry strength were Instron tested with a (3-inch) 7.62 cm sample width, (4 inch) 10.16 cm jaw span at (10 in/min) 25.4 cm/min crosshead speed. The caliper was measured with the TMI tester at 1,99 kPa (0.289 psi). (The only difference in this example from the previous example is the vacuum level and dryer temperature).

| | Example 5 | Example 6 | Example 7 | Example 8 |
|---|---|---|---|---|
| Basis Weight | 13 gm⁻² (gsm) | 18 gm⁻² (gsm) | 24 gm⁻² (gsm) | 30 gm⁻² (gsm) |
| MD grams dry | 1167 | 649 | 1091 | 1605 |
| MD % stretch dry | 1.4 | 3.7 | 4.0 | 5.1 |
| CD grams dry | 630 | 727 | 1130 | 1624 |
| CD % stretch dry | 2.6 | 3.5 | 4.0 | 4.0 |
| GMT grans dry | 857 | 687 | 1110 | 1614 |
| MD grams wet | 393 | 294 | 465 | 671 |
| MD % stratch wet | 1.5 | 5.0 | 5.5 | 5.5 |
| CD grams wet | 223 | 251 | 429 | 586 |
| CD % stretch wet | 2.4 | 3.3 | 3.5 | 3.5 |
| GMT grams wet | 296 | 272 | 447 | 627 |
| MD % wet/dry | 33.7 | 45.3 | 42.6 | 41.8 |
| CD % wet/dry | 35.4 | 34.5 | 38.0 | 36.1 |
| GMT % wet/dry | 34.5 | 40.0 | 40.3 | 38.8 |
| Basis Weight gm⁻² (gsm) | 13.6 | 17.6 | 23.9 | 30.1 |
| 1-Sheet TMI, mm | .335 | .533 | .610 | .655 |
| 10-sheet TMI, mm | 1.94 | 2.91 | 4.00 | 4.55 |
| Bulk cc/g | 24.6 | 30.3 | 25.5 | 21.8 |
| Absorbency @(0.75 psi) | | | | |
| Horizontal g/g | 12.2 | 13.3 | 13.0 | 11.8 |
| 45° g/g | 11.4 | 11.8 | 11.3 | 10.2 |
| Density, gm³(g/cc) | .041 | .033 | .039 | .046 |
| Percent Wet Wrinkle Recovery | 73.8 | 76.7 | 85.0 | 86.7 |

As shown, all examples exhibited high wet resiliency as determined by the Wet Wrinkle Recovery Test.

### Examples 9-11

In ,order to further illustrate this invention, uncreped throughdried tissues were produced using the method substantially as illustrated in Figure 1. More specifically, single-layer, single-ply tissues were made in which all layers comprised unrefined northern softwood bleached chemithermomechanical pulp (BCTMP) fibers. Prior to formation, the BCTMP fibers were pulped for 20 minutes at 4.6 percent consistency and diluted to 2.8 percent consistency after pulping. Kymene 557LX was added at 10-18 kilograms per metric ton of pulp.

A four-layer headbox was used to form the wet web with the unrefined northem softwood BCTMP stock in all four layers. Turbulence-generating inserts recessed about (3 inches) 75 millimeters from the slice and layer dividers extending about (6 inches) 150 millimeters beyond the slice were employed. Flexible lip extensions about (6 inches) 150 millimeters beyond the slice were also used, as taught In U.S. Patent No. 5,129,988 issued July 14, 1992 to Farrington, Jr. and entitled "Extended Flexible Headbox Slice With Parallel Flexible Lip Extensions And Extended Intemal Dividers". The net slice opening was about(0.7.5 inch) 19 millimeters, and water flows in all four headbox layers were comparable. The consistency of the stock fed to the headbox was ranged from about 0.3 to about 0.5 weight percent.

The resulting single-layered sheet was formed on a twin-wire, suction form roll former in which both forming fabrics (12 and 13 in Figure 1) were Asten 866 fabrics. The speed of the forming fabrics ranged from 5.3 to 6.6 meters per second. The newty-formed web was then dewatered to a consistency of about 20-27 percent using vacuum suction from below the forming fabric before being transferred to the transfer fabric, which was traveling from 3.6 to 5.1 meters per second. The resulting rush transfer ranged between 30 percent and 50 percent. The transfer fabric was a Undsay 2164 fabric. A vacuum shoe pulling about (6-15 inches) 150-380 millimeters of mercury vacuum was used to transfer the web to the transfer fabric.

The web was then transferred to a throughdrying fabric (Lindsay Wire T116-3). The throughdrying fabric was traveling at a speed substantially the same as the transfer fabric. The web was carried over a Honeycomb throughdryer operating at a temperature of about (400°F.) 204°C. and dried to final dryness of about 94-98 percent consistency. The resulting uncreped throughdried tissue sheets had the following properties:

| | Example 9 | Example 10 | Example 11 |
|---|---|---|---|
| Kymene, kg/MT | 10 | 18 | 10 |
| Rush Transfer, % | 30 | 30 | 30 |
| Forming Fabric Speed, M/S | 6.6 | 6.6 | 5.9 |
| Transfer fabric Speed, M/S | 5.1 | 5.1 | 4.6 |
| Forming Consistency, % | 0.3 | 0.4 | 0.4 |
| MD grams dry | 4040 | 6340 | 7360 |
| MD % stretch dry | 22.0 | 24.4 | 24.6 |
| CO grams dry | 2940 | 6560 | 4690 |
| CD % stretch dry | 5.3 | 4.0 | 4.7 |
| GMT grams dry | 3446 | 6449 | 5875 |
| MD grams wet | 2702 | 4383 | 3786 |
| MD % stretch wet | 20.5 | 21.5 | 20.5 |
| CD grams wet | 1252 | 2840 | 1917 |
| CD % stretch wet | 6,8 | 4.7 | 5.7 |
| GMT grams wet | 1839 | 3528 | 2694 |
| MD % wet/dry | 66.9 | 69.1 | 51.4 |
| CD % wet/dry | 42.6 | 43.3 | 40.9 |
| GMT % wet/dry | 53.4 | 54.7 | 45.9 |
| Basis Weight(gsm) | 65.2 | 82.8 | 88.8 |
| 1-Sheet TMI mm | 0.899 | 0.884 | 0.950 |
| 10-Sheet TMI mm | 7.01 | 7.21 | 7.89 |
| Bulk 5.17 kPa (cc/g) | 13.8 | 10.7 | 10.7 |
| Absorbency @ (.075 psi) | | | |
| Horizontal g/g | 10.8 | 8.3 | 8.3 |
| 45° g/g | 8.8 | 7.4 | 6.9 |
| Density gm³ (g/cc) | 0.073 | 0.094 | 0.093 |
| Percent wet Wrinkle Recovery | 75.0 | 83.9 | 78.9 |

As shown, all three examples for which the Wet Wrinkle Recovery Test was measured exhibited high wet resiliency as measured by that test.

In order to further illustrate the properties of the absorbent structures of this invention, the wet compressive resiliency properties of some of the foregoing samples were measured and are set forth below.

| Wet Compressive Resiliency | | | | | | | | | |
|---|---|---|---|---|---|---|---|---|---|
| | Example | | | | | | | | |
| | 1 | 2 | 3 | 4 | 6 | 7 | 8 | 9 | 10 |
| Basis Weight, gm⁻²(gsm) | 31.7 | 32.2 | 32.4 | 32.7 | 17.6 | 23.9 | 30.1 | 652 | 82.8 |
| A) Initial Bulk @ 0,17 kPa (0.025 psi) | 18.4 | 18.5 | 19.9 | 21.3 | 28.7 | 22.9 | 21.2 | 15.2 | 12.5 |
| B) Compressed Bulk | 5.2 | 6.0 | 7.1 | 7.9 | 82 | 8.1 | 8.0 | 8.7 | 8.0 |
| C) Final Bulk @ 0,17 kPa (0.025 psi) | 8.4 | 13.6 | 13.0 | 18.0 | 22.7 | 19.3 | 17.7 | 14.1 | 11.4 |
| Compression Ratio (B/A) | 0.28 | 0.32 | 0.36 | 0.37 | 0.286 | 0.345 | 0.378 | 0.571 | 0.639 |
| Springback Ratio (C/A) | 0.46 | 0.73 | 0.66 | 0.85 | 0.791 | 0.841 | 0.838 | 0.929 | 0.917 |
| Loading Energy Ratio | 0.49 | 0.65 | 0.65 | 0.83 | 0.802 | 0.783 | 0.808 | 0.835 | 0.819 |

As shown, the examples of this invention (Examples 4-11) all exhibit high Springback Ratios and high Loading Energy Ratios compared to the controls (Examples 1-3). In addition, some of the examples of this invention also exhibited a high Compressed Bulk of about 7.5 cm³/g (7.5 cc/g)or greater (Examples 9, 10). Also, the examples of this Invention all exhibit Compression Ratios of about 0.7 or less in combination with Springback Ratios of about 0.8 or greater and Loading Energy Ratios of about 0.7 or greater, resulting in a web having a low wet modulus and high wet resiliency.

It will be appreciated that the foregoing examples, given for purposes of illustration, are not to be considered as limiting the scope of this invention which is defined by the following claims.

## Claims

1. A low density tissue or paper towel having a density of about 0.3 gram per cubic centimeter or less, wherein the fibers are Immobilized with wet-resistant bonds, and wherein the wet-resilient low density absorbent structure is uncreped, **characterized In that** the absorbent structure comprises wet-resilient natural fibers, comprised with at least 30 dry weight percent high yield pulp fibers, said tissue or paper towel having a wet : dry geometric mean tensile ratio of about 0.1 or greater, a Compression Ratio of about 0.7 or less and a Springback Ratio of about 0.75 or greater, and a Compressed Bulk of about 7.0 cubic centimeters per gram or greater.

2. The tissue or paper towel of claim 1 having a Wet Wrinkle Recovery of about 60 percent or greater.

3. The tissue or paper towel of claim 1 having a Wet Wrinkle Recovery of about 70 percent or greater.

4. The tissue or paper towel of claim 1 having a Wet Wrinkle Recovery of about 80 percent or greater.

5. The tissue or paper towel of claim 1 having a wet:dry geometric mean tensile strength ratio of about 0.2 or greater.

6. The tissue or paper towel of claim 1 having a wet:dry geometric mean tensile strength ratio of about 0.35 or greater.

7. The tissue or paper towel of claim 1 having a wet:dry geometric mean tensile strength ratio of about 0.5 or greater.

8. The tissue or paper towel of claim 1 having a Compression Ratio of about 0.5 or less.

9. The tissue or paper towel of claim 1 having a Compression Ratio of about 0.6 or less.

10. The tissue or paper towel of claim 1 having a Springback Ratio of about 0.8 or greater.

11. The tissue or paper towel of claim 1 having a Springback Ratio of about 0.9 or greater.

12. The tissue or paper towel of claim 1 having, a Loading Energy Ratio of about 0.7 or greater.

13. The tissue or paper towel of claim 1 having a Loading Energy Ratio of about 0.8 or greater.

14. The tissue or paper towel of claim 1 containing at least about 50 dry weight percent high yield pulp fibers.

15. The tissue or paper towel claim 1 containing about 100 dry weight percent high yield pulp fibers.

16. The tissue or paper towel of claim 1 wherein the high yield pulp fibers are bleached chemithermomachanical pulp fibers.

17. The tissue or paper towel of claim 16 containing at least about 0.2 dry weight percent of a wet strength agent.

18. The tissue or paper towel of claim 16 containing from about 0.1 to about 3 dry weight percent of a wet strength agent.

19. The tissue or paper towel of claim 16 having a density of about 0.1 gram per cubic centimeter or less.

20. The tissue or paper towel of claim 16 having a Compression Ratio of about 0.6 or less and a Springback Ratio of about 0.75 or greater.

21. The tissue or paper towel of claim 16 having a Compression Ratio of ebout 0.5 or less and a Springback Ratio of about 0.75 or greater.

22. The tissue or paper towel of claim 16 having a Compressed Bulk of about 8.0 cubic centimeters per gram or greater.

## Revendications

1. Mouchoir ou serviette en papier de faible masse volumique, ledit mouchoir ou serviette en papier ayant une masse volumique d'environ 0,3 g/cm³ ou moins, dans lequel les fibres sont immobilisées par des liaisons résistantes à l'état humide, et dans lequel la structure absorbante de faible masse volumique, résiliente à l'état humide, est non crêpée, **caractérisé en ce que** la structure absorbante comprend des fibres naturelles résilientes à l'état humide, composées d'au moins 30% en poids sec de fibres de pâte à fort rendement, ledit mouchoir ou serviette en papier ayant un rapport entre les résistances à la traction (moyenne géométrique) humide:sec d'environ 0,1 ou plus, un Rapport de Compression d'environ 0,7 ou moins et un Rapport d'Effet Ressort d'environ 0,75 ou plus et un Indice de Volume Massique à l'Etat Comprimé d'environ 7,0 cm³/g ou plus.

2. Mouchoir ou serviette en papier selon la revendication 1, ayant une Récupération après Froissement à l'Etat Humide d'environ 60% ou plus.

3. Mouchoir ou serviette en papier selon la revendication 1, ayant une Récupération après Froissement à l'Etat Humide d'environ 70% ou plus.

4. Mouchoir ou serviette en papier selon la revendication 1, ayant une Récupération après Froissement à l'Etat Humide d'environ 80% ou plus.

5. Mouchoir ou serviette en papier selon la revendication 1, ayant un rapport entre les résistances à la traction (moyenne géométrique) humide:sec d'environ 0,2 ou plus.

6. Mouchoir ou serviette en papier selon la revendication 1, ayant un rapport entre les résistances à la traction (moyenne géométrique) humide:sec d'environ 0,35 ou plus.

7. Mouchoir ou serviette en papier selon la revendication 1, ayant un rapport entre les résistances à la traction (moyenne géométrique) humide:sec d'environ 0,5 ou plus.

8. Mouchoir ou serviette en papier selon la revendication 1, ayant un Rapport de Compression d'environ 0,5 ou moins.

9. Mouchoir ou serviette en papier selon la revendication 1, ayant un Rapport de Compression d'environ 0,6 ou moins.

10. Mouchoir ou serviette en papier selon la revendication 1, ayant un Rapport d'Effet Ressort d'environ 0,8 ou plus.

11. Mouchoir ou serviette en papier selon la revendication 1, ayant un Rapport d'Effet Ressort d'environ 0,9 ou plus.

12. Mouchoir ou serviette en papier selon la revendication 1, ayant un Rapport d'Energie de Charge d'environ 0,7 ou plus.

13. Mouchoir ou serviette en papier selon la revendication 1, ayant un Rapport d'Energie de Charge d'environ 0,8 ou plus.

14. Mouchoir ou serviette en papier selon la revendication 1, contenant au moins environ 50% en poids sec de fibres de pâte à fort rendement.

15. Mouchoir ou serviette en papier selon la revendication 1, contenant environ 100% en poids sec de fibres de pâte à fort rendement.

16. Mouchoir ou serviette en papier selon la revendication 1, dans lequel les fibres de pâte à fort rendement sont des fibres de pâte chimico-thermomécanique blanchie.

17. Mouchoir ou serviette en papier selon la revendication 16, contenant au moins environ 0,2% en poids sec d'un agent augmentant la résistance à l'état humide.

18. Mouchoir ou serviette en papier selon la revendication 16, contenant d'environ 0,1 à environ 3% en poids sec d'un agent augmentant la résistance à l'état humide.

19. Mouchoir ou serviette en papier selon la revendication 16, ayant une masse volumique d'environ 0,1 g/cm³ ou moins.

20. Mouchoir ou serviette en papier selon la revendication 16, ayant un Rapport de Compression d'environ 0,6 ou moins et un Rapport d'Effet Ressort d'environ 0,75 ou plus.

21. Mouchoir ou serviette en papier selon la revendication 16, ayant un Rapport de Compression d'environ 0,5 ou moins et un Rapport d'Effet Ressort d'environ 0,75 ou plus.

22. Mouchoir ou serviette en papier selon la revendication 16, ayant un Indice de Volume Massique à l'Etat Comprimé d'environ 8,0 cm³/g ou plus.

## Patentansprüche

1. Niedrigdichtes Tissue oder Papierhandtuch, wobei das Tissue oder Papierhandtuch eine Dichte von etwa 0,3 Gramm pro Kubikzentimeter oder weniger aufweist, wobei die Fasern durch nassresistente Verbindungen bewegungsunfähig sind, und wobei die nasselastische, niedrigdichte, absorbierende Struktur ungekreppt ist, **dadurch gekennzeichnet, dass** die absorbierende Struktur nasselastische Naturfasern umfasst, die wenigstens 30 Trockengewichtsprozent an hochergiebigen Zellstofffasern umfassen, wobei das Tissue oder Papierhandtuch einen geometrischen Mittelwert des Nass:Trocken-Zugverhältnisses von etwa 0,1 oder mehr, ein Kompressionsverhältnis von etwa 0,7 oder weniger und ein Rückfederverhältnis von etwa 0,75 oder mehr und ein Pressvolumen (Compressed Bulk) von etwa 7,0 Kubikzentimeter pro Gramm oder mehr aufweist.

2. Tissue oder Papierhandtuch gemäß Anspruch 1, das eine Nassknitterregenerierung von etwa 60 Prozent oder mehr aufweist.

3. Tissue oder Papierhandtuch gemäß Anspruch 1, das eine Nassknitterregenerierung von etwa 70 Prozent oder mehr aufweist.

4. Tissue oder Papierhandtuch gemäß Anspruch 1, das eine Nassknitterregenerierung von etwa 80 Prozent oder mehr aufweist.

5. Tissue oder Papierhandtuch gemäß Anspruch 1, das einen geometrischen Mittelwert des Nass:Trocken-Zugfestigkeitsverhältnisses von etwa 0,2 oder mehr aufweist.

6. Tissue oder Papierhandtuch gemäß Anspruch 1, das einen geometrischen Mittelwert des Nass-Trocken-Zugfestigkeitsverhältnisses von etwa 0,35 oder mehr aufweist.

7. Tissue oder Papierhandtuch gemäß Anspruch 1, das einen geometrischen Mittelwert des Nass-Trocken-Zugfestigkeitsverhältnisses von etwa 0,5 oder mehr aufweist.

8. Tissue oder Papierhandtuch gemäß Anspruch 1, das ein Kompressionsverhältnis von etwa 0,5 oder weniger aufweist.

9. Tissue oder Papierhandtuch gemäß Anspruch 1, das ein Kompressionsverhältnis von etwa 0,6 oder weniger aufweist.

10. Tissue oder Papierhandtuch gemäß Anspruch 1, das ein Rückfederverhältnis von etwa 0,8 oder mehr aufweist.

11. Tissue oder Papierhandtuch gemäß Anspruch 1, das ein Rückfederverhältnis von etwa 0,9 oder mehr aufweist.

12. Tissue oder Papierhandtuch gemäß Anspruch 1, das ein Ladeenergieverhältnis von etwa 0,7 oder mehr aufweist.

13. Tissue oder Papierhandtuch gemäß Anspruch 1, das ein Ladeenergieverhältnis von etwa 0,8 oder mehr aufweist.

14. Tissue oder Papierhandtuch gemäß Anspruch 1, das wenigstens etwa 50 Trockengewichtsprozent an hochergiebigen Zellstofffasern umfasst.

15. Tissue oder Papierhandtuch gemäß Anspruch 1, das etwa 100 Trockengewichtsprozent an hochergiebigen Zellstofffasern umfasst.

16. Tissue oder Papierhandtuch gemäß Anspruch 1, wobei die hochergiebigen Zellstofffasern gebleichte chemothermomechanische Zellstofffasern sind.

17. Tissue oder Papierhandtuch gemäß Anspruch 16, das wenigstens etwa 0,2 Trockengewichtsprozent eines Nassstärkenwirkstoffs umfasst.

18. Tissue oder Papierhandtuch gemäß Anspruch 16, das etwa 0,1 bis etwa 3 Trockengewichtsprozent eines Nassstärkenwirkstoffs umfasst.

19. Tissue oder Papierhandtuch gemäß Anspruch 16, das eine Dichte von etwa 0,1 Gramm pro Kubikzentimeter oder weniger aufweist.

20. Tissue oder Papierhandtuch gemäß Anspruch 16, das ein Kompressionsverhältnis von etwa 0,6 oder weniger und ein Rückfederverhältnis von etwa 0,75 oder mehr aufweist.

21. Tissue oder Papierhandtuch gemäß Anspruch 16, das ein Kompressionsverhältnis von etwa 0,5 oder weniger und ein Rückfederverhältnis von etwa 0,75 oder mehr aufweist.

22. Tissue oder Papierhandtuch gemäß Anspruch 16, das ein Pressvolumen von etwa 8,0 Kubikzentimeter pro Gramm oder mehr aufweist.
